# EUROPEAN PATENT APPLICATION

(11) **EP 4 570 277 A1**
(43) Date of publication of application: **18.06.2025**
(21) Application number: 23851509.2
(22) Date of filing: 05.07.2023
(51) Int. Cl.: A61L 27/36, A61L 27/56, A61L 27/58

(54) **SCLERA REINFORCEMENT MATERIAL, METHOD FOR PREPARING SAME AND USE THEREOF**

(30) Priority: 08.08.2022 CN 202210946834
(71) Applicant: Neo Modulus (Suzhou) Medical SCI-Tech Co., Ltd., Suzhou, Jiangsu 215163 (CN)
(72) Inventor: CHEN, Weiming, Suzhou, Jiangsu 215163 (CN); LIU, Changjun, Suzhou, Jiangsu 215163 (CN); LI, Xiaohong, Suzhou, Jiangsu 215163 (CN)
(74) Representative: Haseltine Lake Kempner LLP
(86) International application number: PCT/CN2023/105874
(87) International publication number: WO 2024/032284

(57) **Abstract**

The present invention relates to a scleral reinforcement material, its preparation method, and its uses. The preparation method comprises the following steps: (1) providing animal skin tissue as raw material and isolating the dermal tissue therefrom; (2) subjecting the dermal tissue to degreasing and decellularization; and (3) cross-linking the decellularized dermal tissue to achieve an elastic modulus of 15 MPa and 50 MPa and a degradation rate of less than 20% over a period of 6 to 12 months. The scleral reinforcement material of the present invention exhibits both mechanical and degradation properties that are highly suitable for scleral reinforcement.

## Description

### CROSS-REFERENCE TO RELATED APPLICATIONS

This application claims the benefit and priority of Chinese Patent Application No. 202210946834.6, filed on August 8, 2022, entitled *"A Scleral Reinforcement Material, Its Preparation Method, and Use, "* the contents of which are incorporated herein by reference in their entirety.

### TECHNICAL FIELD

The present invention relates to the field of medical biomaterials, specifically to a scleral reinforcement material, its preparation method, and its use.

### BACKGROUND

Scleral reinforcement is a preventive surgical procedure that involves placing a reinforcing material (such as a patch) at the posterior pole and macular region, ensuring tight adhesion to the recipient sclera. This procedure promotes neovascularization, leading to the formation of a thickened new sclera. Mechanically reinforcing the eye wall helps control ocular expansion, thereby alleviating posterior elongation and stabilizing diopter.

In recent years, with advancements in medical technology, scleral reinforcement materials have undergone continuous optimization and improvement. Ophthalmic patches used for scleral reinforcement can be categorized into synthetic materials, autologous materials, allogeneic materials, and xenogeneic materials. Synthetic materials include artificial pericardial patches and polytetrafluoroethylene. While these materials offer low elasticity, certain flexibility, plasticity, and stable physical and chemical properties, they exhibit poor tissue compatibility, do not integrate with the recipient's tissue, and hinder neovascularization. And they fail to improve the blood supply to the recipient tissue. Postoperative complications such as effusion, hematoma, rejection, deformation, displacement, and prolapse are common. Autologous materials include autologous fascia lata and autologous dermis. These materials provide excellent tissue compatibility, eliminate the risk of rejection, and possess strong anti-infection properties. However, their availability is limited, they require additional surgical trauma for harvesting, and they pose risks of intraoperative and postoperative complications. Allogeneic materials such as allogeneic sclera and dura mater offer good tissue tolerance and mild postoperative reactions. However, they are difficult to source and preserve, and they are prone to rejection, infection, and disease transmission. Xenogeneic materials primarily comprise acellular dermal matrix. This natural biomaterial is derived from mammalian skin using physical and/or chemical decellularization techniques to remove cellular components and skin appendages while preserving the three-dimensional scaffold structure of the dermal matrix.

Although acellular dermal matrix materials offer several advantages, including abundant availability and excellent biocompatibility, conventional acellular dermal matrices exhibit excessively high elongation at break, making them unsuitable for scleral reinforcement. Effective scleral reinforcement requires materials with lower elongation to prevent scleral elongation. Moreover, conventional acellular dermal matrices degrade rapidly, typically within about 1 to 3 months, whereas a longer duration is necessary to ensure sustained reinforcement. Consequently, existing acellular dermal matrix materials are not well-suited for scleral reinforcement applications.

### SUMMARY OF THE INVENTION

One objective of the present invention is to provide a method for preparing a scleral reinforcement material that overcomes the limitations in prior arts. The method comprises the following steps:
(1) providing animal skin tissue as raw material and isolating the dermal tissue therefrom;
(2) subjecting the dermal tissue to degreasing and decellularization; and
(3) cross-linking the decellularized dermal tissue to achieve an elastic modulus of 15 MPa to 50 MPa and a degradation rate of less than 20% over 6 to 12 months.

In some embodiments of the present invention, the cross-linking in step (3) comprises chemical and/or thermal cross-linking.

In some embodiments of the present invention, the chemical cross-linking agent is selected from one or more of glutaraldehyde, genipin, carbodiimide, and epoxy compounds.

In some embodiments of the present invention, the carbodiimide comprises an EDC (1-(3-dimethylaminopropyl)-3-ethylcarbodiimide hydrochloride) aqueous solution with a weight concentration of 1 wt% to 10 wt%.

In some embodiments of the present invention, the chemical cross-linking is performed at a temperature of 0°C to 37°C or 4°C to 25°C for 12 to 60 hours or 24 to 48 hours.

In some embodiments of the present invention, the cross-linking may be conducted via immersion or fumigation in a cross-linking agent.

In some embodiments of the present invention, after chemical cross-linking, an additional step may be included to remove residual cross-linking agents through one or more of the following methods: water washing, ventilation drying, or high-temperature drying.

In some embodiments of the present invention, the elastic modulus of the cross-linked material is in the range of 30 MPa to 50 MPa.

In some embodiments of the present invention, the decellularized dermal tissue is cross-linked to achieve a degradation rate of less than 5% over 26 weeks.

In some embodiments of the present invention, the decellularized dermal tissue is cross-linked to achieve an elongation at break of 20% to 60% or 20% to 30%.

In some embodiments of the present invention, the animal skin tissue used in the method is derived from cattle, pigs, sheep or horses.

In some embodiments of the present invention, the degreasing and decellularization in step (2) is performed using a salt solution, base solution, acid solution, or alcohol solution.

The salt solution comprises one or more of disodium hydrogen phosphate, dipotassium hydrogen phosphate, sodium chloride, and potassium chloride.

The base solution comprises one or more of sodium hydroxide, potassium hydroxide, calcium hydroxide, and sodium carbonate.

The acid solution comprises one or more of hydrochloric acid, acetic acid, and carbonic acid.

The alcohol solution comprises one or more of methanol, ethanol, ethylene glycol, and isopropanol.

In some embodiments of the present invention, the method further comprises drying the cross-linked, decellularized dermal tissue, followed by sterilization, wherein the drying includes one or more of ventilation drying, forced air drying, organic solvent drying, and vacuum drying; and the sterilization includes electron beam sterilization, irradiation sterilization, ethylene oxide sterilization, or low-temperature ion sterilization.

In some embodiments of the present invention, the preparation method further comprises drying the decellularized dermal tissue to obtain a dry sample, and packaging the dried sample and performing final sterilization.

Another objective of the invention is to provide a sclera reinforcement material prepared according to the method of the invention.

In some embodiments, the scleral reinforcement material prepared according to the method of the invention is a white or slightly yellow flake, primarily comprised of collagen with trace amounts of glycosaminoglycans. The method effectively removes immunogenic components, such as residual animal cells, ensuring that the residual DNA content is less than 20 ng/mg.

A further objective of the invention is to provide the use of scleral reinforcement material in scleral reinforcement surgery.

Compared to prior arts, the present invention offers the following technical advantages:
(1) The preparation method of the scleral reinforcement material utilizes animal dermal tissue as a raw material. Through degreasing, decellularization, and cross-linking, the resulting material exhibits mechanical properties well-suited for scleral reinforcement. Specifically, it achieves an optimal balance of elastic modulus and elongation, ensuring effective reinforcement and prevention of scleral elongation. The processed dermal tissue becomes stronger, with reduced elongation and resistance to excessive stretching. Simultaneously, after hydration, the material retains sufficient softness for comfortable implantation in the sclera, addressing the issue of traditional dermal materials that become excessively rigid as their hardness increases, leading to ocular discomfort.
(2) The scleral reinforcement material exhibits a controlled degradation rate suitable for scleral reinforcement, superior to conventional acellular dermal matrices, which degrade completely over about 1 to 3 months. In contrast, the material of the invention remains in the scleral tissue for 6 to 12 months, allowing sufficient time for new tissue regeneration before being gradually absorbed by the body. This controlled degradation prevents premature loss of reinforcement, which could otherwise compromise the surgical outcome, while also avoiding prolonged inflammation or foreign body reactions associated with excessively slow degradation.
(3) The scleral reinforcement material features a porous architecture that promotes cellular growth. Once implanted into the sclera, the material facilitates fusion with the surrounding tissue, leading to the formation of thicker, reinforced scleral tissue over time.
(4) Derived from animal dermal tissue, the scleral reinforcement material demonstrates high biocompatibility and undergoes complete degradation into small-molecule amino acids that can be naturally absorbed by the body.

The decellularization process effectively removes immunogenic components, minimizing the risk of immune rejection post-implantation. Furthermore, the process avoids the use of toxic or potentially immunogenic reagents, ensuring the biological safety of the material.

The material has a soft texture, preventing any interference with ocular movement or the sensation of a foreign body after implantation. Additionally, the cross-linking treatment enhances the mechanical strength of the dermal tissue, making it suitable for scleral reinforcement across different degrees of myopia.

The preparation method supports standardized batch processing, facilitating widespread clinical adoption and enabling broader accessibility for patients requiring scleral reinforcement surgery.

The present invention will be described in further detail with reference to the accompanying figures and illustrative embodiments.

### BRIEF DESCRIPTION OF THE FIGURES

FIG. 1 is a photograph illustrating the appearance of the scleral reinforcement material prepared in Example 1 of the present invention.
FIG. 2 is scanning electron microscope (SEM) image depicting the surface morphology of the scleral reinforcement material from Example 1.
FIG. 3 is hematoxylin and eosin (HE) stained image (400× magnification) of the scleral reinforcement material after decellularization, as described in Example 1.
FIG. 4 is a comparative chart displaying the residual cellular DNA content in the dermal raw material versus the scleral reinforcement material used in Example 1.
FIG. 5 is a graphical representation illustrating the degradation rates of the scleral reinforcement materials prepared in Examples 1 to 6 after enzymatic hydrolysis in a collagenase solution.
FIG. 6 is HE-stained images of tissue sections showing the histological response to the scleral reinforcement material from Example 4, implanted subcutaneously in rabbits for 1 week, 4 weeks, 8 weeks, 12 weeks, and 26 weeks.
FIG. 7 is a photograph of the scleral reinforcement material from Example 4, showing its appearance after implantation in a rabbit's sclera for 3 months.

### DETAILED DESCRIPTION

The technical solution of the invention will be clearly and comprehensively described below in conjunction with its embodiments and accompanying figures. The described embodiments are only illustrative and represent part of the possible embodiments, rather than an exhaustive enumeration of all embodiments. The following description of at least one exemplary embodiment serves purely as an illustration and should not be construed as a limitation to the method of the invention. Based on the illustrative embodiments, all other embodiments that can be conceived by those skilled in the art without requiring inventive effort fall within the scope of protection of the invention.

Unless explicitly stated otherwise, the numerical values provided in these embodiments do not limit the scope of the invention. Techniques and methods well known to those skilled in the art may not be discussed in detail; however, where appropriate, they should be considered as a part of this specification. In all examples presented and discussed herein, any specific values should be interpreted as exemplary rather than restrictive. Accordingly, other exemplary embodiments may feature different values.

### Example 1:

The scleral reinforcement material in this example was prepared using the following steps:
(1) Pig skin was selected as the raw material. The skin was thoroughly washed, and a dermal tissue sheet with a uniform thickness of 1 mm was excised from the subcutaneous fascia layer of the skin, retaining both the papillary layer and the reticular layer.
(2) The excised dermal tissue was subjected to a degreasing and decellularization treatment as follows:
   ① The dermal tissue was cleaned with a 0.5 wt% K₂HPO₄-KH₂PO₄ salt solution for 4 hours, then soaked in 4 wt% sodium hydroxide at 10°C to 25°C, stirred for 5 hours, and rinsed with purified water for 4 hours.
   ② The treated tissue from step ① was then immersed in a 1 wt% hydrochloric acid aqueous solution at 10°C to 25°C, stirred for 5 hours, and rinsed again with purified water for 4 hours.
   ③ Steps ① and ② were repeated 2 to 4 times to ensure complete removal of cellular components.
   ④ The tissue sheet obtained from step ③ was washed with anhydrous ethanol for 24 hours, followed by an additional rinse with purified water for 4 hours.
   ⑤ The tissue sheet obtained from step ④ was then freeze-dried for 4 to 8 hours to remove residual moisture.
(3) The tissue sheet obtained from step ⑤ was subjected to a cross-linking treatment as follows:
   The freeze-dried sheet was immersed in a 1 wt% EDC aqueous solution at 4°C for 48 hours, followed by a thorough rinse with purified water for 4 hours.
(4) The cross-linked tissue sheet obtained from step (3) was placed in a vacuum freeze dryer and freeze-dried for 48 hours.
(5) The sheet obtained from step (4) was packaged in a blister box and sterilized using ethylene oxide, producing the scleral reinforcement material, which is designated as Sample No. 2.

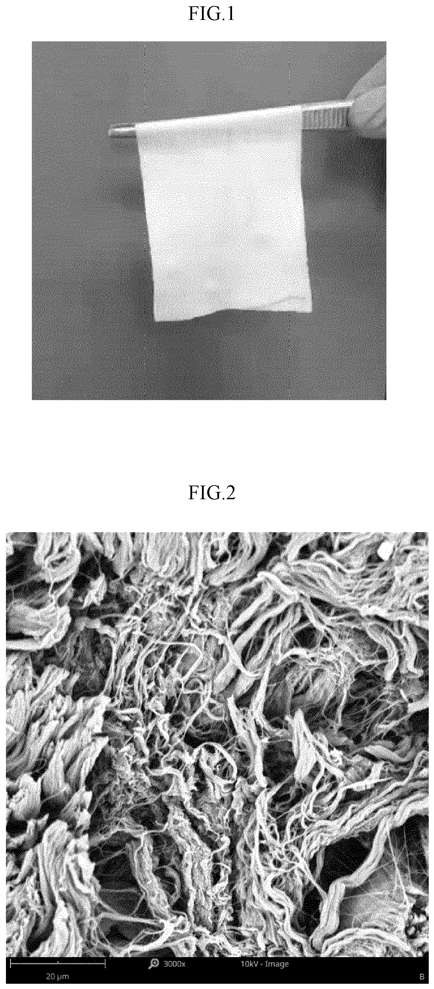

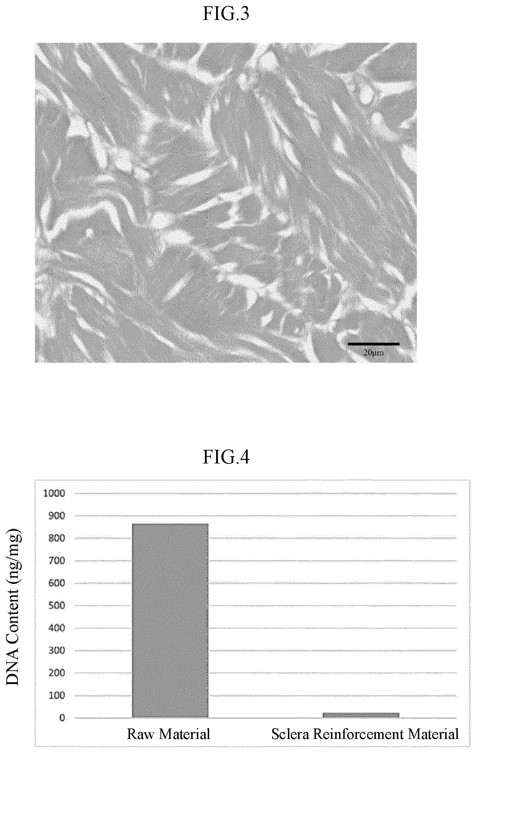

The appearance of the produced scleral reinforcement material is shown in FIG. 1. The surface morphology, as observed under a scanning electron microscope (SEM), is shown in FIG. 2. The HE-stained image (400× magnification) of the decellularized tissue is shown in FIG. 3.

The residual DNA content in the raw dermal tissue and the final scleral reinforcement material is compared in FIG. 4. According to FIG. 4, the residual DNA content in the decellularized dermal tissue is minimal, with a decellularization efficiency exceeding 98%. Additionally, ccompared to traditional dermis decellularization methods that use surfactants (e.g., SDS) or enzymes, the approach in this invention, which utilizes salt, acid, or base solutions, is significantly less toxic, easier to remove, and leaves no harmful chemical residues, making it a safer decellularization method. Surfactants such as SDS are highly toxic, genetically harmful, and can degrade material structures, making complete removal challenging. Enzymatic methods, on the other hand, can compromise the material's structural integrity and strength while also potentially triggering immune rejection reactions.

### Example 2

The difference between Example 2 and Example 1 is that the concentration of the crosslinking agent EDC aqueous solution is 2.5 wt%, while all other conditions remain the same as those in Example 1. The scleral reinforcement material obtained in Example 2 is designated as Sample No. 3.

### Example 3

The difference between Example 3 and Example 1 is that the concentration of the crosslinking agent EDC aqueous solution is 5 wt%, while all other conditions remain the same as those in Example 1. The scleral reinforcement material obtained in Example 3 is designated as Sample No. 4.

### Example 4

The scleral reinforcement material in this example was prepared using the following steps:
(1) Pig skin was selected as the raw material. The skin was thoroughly washed, and a dermal tissue sheet with a uniform thickness of 0.6 mm was excised from the subcutaneous fascia layer of the skin, retaining both the papillary layer and the reticular layer.
(2) The excised dermal tissue was subjected to a degreasing and decellularization treatment as follows:
   ① The dermal tissue was cleaned with a 0.9 wt% sodium chloride solution for 2 hours, then soaked in 4 wt% sodium hydroxide at 10°C to 25°C, stirred for 5 hours, and rinsed with purified water for 4 hours.
   ② The treated tissue sheet from step ① was then immersed in a 1 wt% hydrochloric acid aqueous solution at 10°C to 25°C, stirred for 5 hours, and rinsed again with purified water for 4 hours.
   ③ Steps ① and ② were repeated 2 to 4 times to ensure complete removal of cellular components.
   ④ The tissue sheet obtained from step ③ was washed with anhydrous ethanol for 24 hours, followed by an additional rinse with purified water for 4 hours.
   ⑤ The tissue sheet obtained from step ④ was then freeze-dried for 48 hours to remove residual moisture.
(3) The tissue sheet obtained from step ⑤ was subjected to a cross-linking treatment as follows:
   The freeze-dried sheet was immersed in a 5 wt% EDC aqueous solution at 25°C for 48 hours, followed by a thorough rinse with purified water for 4 hours.
(4) The cross-linked tissue sheet obtained from step (3) was placed in a vacuum freeze dryer and freeze-dried for 48 hours.
(5) The sheet obtained from step (4) was packaged in a blister box and sterilized using electron beam irradiation, producing the scleral reinforcement material, which is designated as Sample No. 7.

### Example 5

The difference between this example and Example 4 is that the concentration of the crosslinking agent EDC aqueous solution is 2.5 wt%, while all other conditions remain the same as those in Example 4. The scleral reinforcement material obtained in Example 5 is designated as Sample No. 6.

### Example 6

This example differs from Example 4 in that the concentration of the EDC aqueous solution used for cross-linking is 1 wt%, while all other conditions remain the same as in Example 4. The scleral reinforcement material obtained in Example 6 is designated as Sample No. 5.

The scleral reinforcement materials prepared in Examples 1-6 (corresponding to Samples 2, 3, 4, 7, 6, and 5 respectively) were tested, with a dermal material (Sample No. 1) that was not cross-linked after decellularization serving as a control.

### 1. Test of Elastic Modulus and Elongation at Break

The scleral reinforcement materials prepared in Examples 1-6 were cut into dimensions of 50 mm × 10 mm and soaked in purified water for 10 minutes. The thickness of the sample was measured at three points within the gauge length using a thickness gauge, and the average thickness was recorded. The sample was then placed in the tensile machine clamp, stretched, and the distance between the upper and lower clamps was measured. The tensile machine was set to a speed of 25 mm/min. The resulting values for elastic modulus and elongation at break are presented in Table 1

**Table 1**

| Sample No. | Crosslinking agent | Temperature | Elastic modulus (MPa) | Elongation at break (%) |
|---|---|---|---|---|
| 1 | 0% | / | 11.7 | 83.59 |
| 2 | 1% | 4 °C | 16.85 | 56.9 |
| 3 | 2.50% | 4 °C | 21.08 | 49.3 |
| 4 | 5% | 4 °C | 23.05 | 44.95 |
| 5 | 1% | 25 °C | 36.29 | 26.15 |
| 6 | 2.50% | 25 °C | 42.35 | 24.38 |
| 7 | 5% | 25 °C | 47.56 | 20.71 |

As shown in Table 1, the scleral reinforcement material obtained through the cross-linking treatment of the present invention demonstrates a significant difference when compared to the non-cross-linked control sample (Sample 1). The elastic modulus of the scleral reinforcement material increases with higher concentrations of the cross-linking agent and increased cross-linking temperature, while the elongation at break decreases under the same conditions. Based on the findings of this study, it is evident that the scleral reinforcement materials prepared in Examples 1 to 6 meet the mechanical property requirements for scleral reinforcement. Specifically, when the elastic modulus of the material ranges from 30 MPa to 50 MPa, and the elongation at break is between 20% and 30%, the material is particularly well-suited for scleral reinforcement applications.

### 2. In Vitro Degradation Performance Test

An accelerated enzymatic degradation test was conducted using a collagenase solution to evaluate the degradation performance of the scleral reinforcement materials from Examples 1 to 6, as well as the non-crosslinked materials.

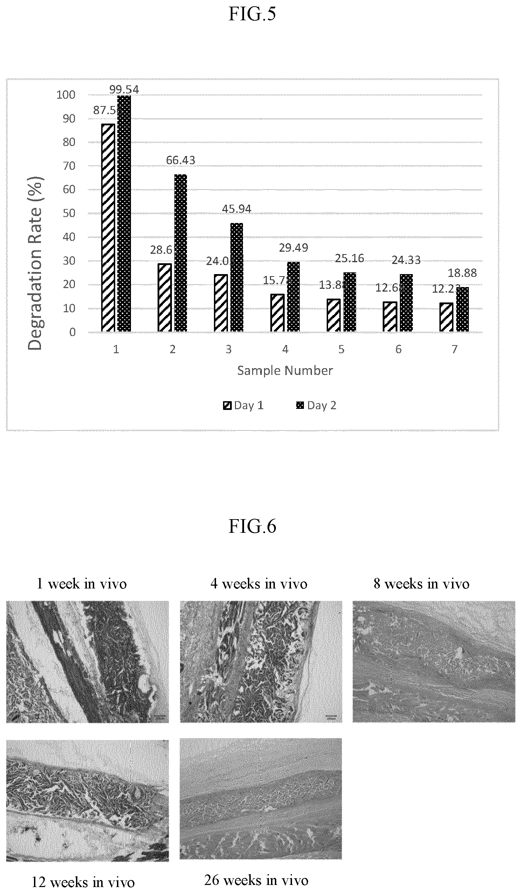

The scleral reinforcement material samples from Examples 1 to 6 and the non-crosslinked control sample were cut to a size of 1 cm × 1 cm and placed in vials containing a type I collagenase solution (concentration: 100 U/mL). The vials were then placed in a 37°C water bath. After 1 day and 2 days of degradation, the samples were freeze-dried, weighed, and the degradation rate for each sample was calculated. The results are shown in Figure 5. As illustrated in Figure 5, the degradation rate of the scleral reinforcement materials from Examples 1 to 6 is significantly lower than that of the non-crosslinked control sample. Specifically, after 1 day and 2 days of accelerated enzymatic hydrolysis in the collagenase solution, the degradation rate of the non-crosslinked control sample was as high as 87.56% and 99.54%, respectively. In contrast, the degradation rate of the scleral reinforcement material samples from Examples 1 to 6 was notably lower. When applied in vivo for scleral reinforcement, the degradation rate of the scleral reinforcement materials of the invention is expected to be much lower than that observed during collagenase-accelerated enzymatic hydrolysis. This lower degradation rate will be further confirmed in the subsequent animal subcutaneous implantation degradation test.

### 3. Animal Subcutaneous Implantation Degradation Test

To evaluate the in vivo degradation performance, the scleral reinforcement material prepared in Example 4 was tested through subcutaneous implantation in animals.

The degradation test was conducted with cycles at 1 week, 4 weeks, 8 weeks, 12 weeks, and 26 weeks. A total of 20 healthy rabbits were selected, with 3 rabbits assigned to each test cycle. For subcutaneous implantation, the rabbits were anesthetized via intraperitoneal injection of sodium pentobarbital at a dose of 40 mg/kg, using a 1% mass concentration of the anesthetic. During anesthesia, special care was taken to maintain the animals' body temperature to prevent hypothermia. The rabbits were placed on the operating table, their fur was shaved along both sides of the spine, and the skin in the shaved area was disinfected. The surgical area was approximately 4 cm x 4 cm. Following the implantation protocol, a 1 cm skin incision was made at each implantation site on both sides of the spine, ensuring at least 1 cm between the incisions. Subcutaneous tissue was bluntly separated down to the superficial fascia and muscle layer, creating four subcutaneous pockets on each side of the rabbit. The test material was implanted on one side, and the incisions were closed and sutured in layers, with the wound disinfected using iodine. The test periods were 1 week, 4 weeks, 8 weeks, 12 weeks, and 26 weeks. Tissue samples from each time point were collected and sliced for analysis of degradation in the scleral reinforcement material. The HE-stained tissue slices from the various time points (1 week, 4 weeks, 8 weeks, 12 weeks, and 26 weeks) are shown in Figure 6.

As shown in Figure 6, after 1, 4, 8, and 12 weeks of implantation, the scleral reinforcement material had not degraded. Even after 26 weeks, the material maintained its complete structure, with a degradation rate of less than 5%. This demonstrates that the scleral reinforcement material of the present invention exhibits good resistance to degradation in vivo, meeting the performance requirements for scleral reinforcement.

### 4. Test of Scleral Reinforcement in Animal

The scleral reinforcement material prepared in Example 4 was tested in a rabbit to evaluate its reinforcement performance.

The rabbit was placed in a supine position on the operating table under sterile conditions, and anesthesia was induced through intramuscular injection of Su Mian Xin. A 360° incision was made at the corneal and conjunctival margins using ophthalmic scissors, with an additional incision of approximately 6 mm in length at the upper and lower parts. The conjunctival sac was bluntly dissected to fully expose the sclera. The external, superior, and inferior rectus muscles were hooked with an oblique hook, and traction lines were created for these muscles. The scleral reinforcement material trimmed to 25 mm × 8 mm was then passed under the superior, external, and inferior rectus muscles. The acellular dermis strip was adjusted so that its central portion was positioned at the posterior pole, closely adhering to the sclera. Both ends of the strip were fixed to the nasal side of the upper and lower rectus muscle attachments. The upper and lower conjunctival incisions were sutured with absorbable sutures, and the operated eye was bandaged with sterile dressings.

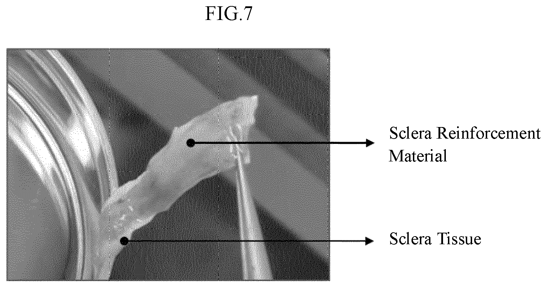

Three months post-operation, the animals were euthanized by air embolization via the ear vein. The eyes were removed aseptically, and the cornea, iris, lens, vitreous body, choroid, and retinal tissues were excised. The sclera was washed with physiological saline, and the sclera, along with the attached reinforcement material, was sectioned according to the size of the reinforcement strip in the experimental eye and photographed for observation. The fusion between the sclera and the scleral reinforcement material is shown in Figure 7. As depicted, the scleral reinforcement material of the embodiment has fused well with the sclera of the test animal. The strength of the fused tissue exceeds that of the sclera prior to reinforcement, demonstrating the successful reinforcement effect.

The present invention has been described in detail above with reference to exemplary embodiments. These embodiments are intended for illustrative purposes only and should not be construed as limiting the scope of protection of the invention. Those skilled in the art may make various modifications, alterations, or replacement without departing from the spirit of the invention. Therefore, all such equivalent modifications made in accordance with the principles of the invention fall within the scope of the invention as defined by the claims.

## Claims

1. A method for preparing a sclera reinforcement material, comprising:
(1) providing animal skin tissue as raw material and isolating the dermal tissue therefrom;
(2) subjecting the dermal tissue to degreasing and decellularization; and
(3) cross-linking the decellularized dermal tissue to achieve an elastic modulus of 15 MPa to 50 MPa and a degradation rate of less than 20% over 6 to 12 months.

2. The method of claim 1, wherein the cross-linking in step (3) comprises chemical and/or thermal cross-linking.

3. The method of claim 2, wherein the chemical cross-linking agent is selected from one or more of glutaraldehyde, genipin, carbodiimide, and epoxy compounds.

4. The method of claim 3, wherein the carbodiimide comprises an EDC aqueous solution with a weigh concentration of 1 wt% to 10 wt% or 1 wt% to 5 wt%.

5. The method of claim 3 or 4, wherein the chemical cross-linking is performed at a temperature of 0°C to 37°C or 4°C to 25°C for 12 to 60 hours.

6. The method of claim 1, wherein the elastic modulus is 30 MPa to 50 MPa.

7. The method of claim 1, wherein the cross-linked, decellularized dermal tissue has an elongation at break of 20% to 60% or 20% to 30%.

8. The method of claim 1, wherein the degreasing and decellularization in step (2) is performed using a salt solution, base solution, acid solution, or alcohol solution, wherein:
the salt solution comprises one or more of disodium hydrogen phosphate, dipotassium hydrogen phosphate, sodium chloride, and potassium chloride;
the base solution comprises one or more of sodium hydroxide, potassium hydroxide, calcium hydroxide, and sodium carbonate;
the acid solution comprises one or more of hydrochloric acid, acetic acid, and carbonic acid; and
the alcohol solution comprises one or more of methanol, ethanol, ethylene glycol, and isopropanol.

9. The method of claim 1, further comprising drying the cross-linked, decellularized dermal tissue, followed by sterilization, wherein:
the drying includes one or more of ventilation drying, forced air drying, organic solvent drying, and vacuum drying; and
the sterilization includes electron beam sterilization, irradiation sterilization, ethylene oxide sterilization, or low-temperature ion sterilization.

10. A sclera reinforcing material prepared according to the method of any one of claims 1 to 9.

11. Use of the scleral reinforcement material in scleral reinforcement surgery, wherein the material is prepared according to the method of any one of claims 1 to 9.
